(19) Europäisches Patentamt
European Patent Office
Office européen des brevets

(11) EP 1 091 942 B1

(12) EUROPEAN PATENT SPECIFICATION

(45) Date of publication and mention of the grant of the patent: **30.03.2005 Bulletin 2005/13**

(21) Application number: **99931019.6**

(22) Date of filing: **01.07.1999**

(51) Int Cl.[7]: **C07D 235/30**, C07D 417/04, C07D 403/10, C07D 417/12, C07D 219/08, C07D 255/04, C07D 239/42, C07D 487/18, C07D 235/20, C07D 279/18, A61K 31/415, A61K 31/425, A61K 31/435, A61K 31/395, A61K 31/505, A61K 31/155

(86) International application number: **PCT/DK1999/000378**

(87) International publication number: **WO 2000/001676 (13.01.2000 Gazette 2000/02)**

(54) **POTASSIUM CHANNEL BLOCKING AGENTS**

KALIUMKANAL-BLOCKIERENDE MITTEL

AGENTS DE BLOCAGE DES CANAUX A POTASSIUM

(84) Designated Contracting States:
**AT BE CH CY DE DK ES FI FR GB GR IE IT LI LU MC NL PT SE**

(30) Priority: **02.07.1998 DK 86598**

(43) Date of publication of application: **18.04.2001 Bulletin 2001/16**

(73) Proprietor: **NEUROSEARCH A/S**
**2750 Ballerup (DK)**

(72) Inventors:
- **TEUBER, Lene**
  **DK-2750 Ballerup (DK)**
- **OLESEN, S ren-Peter**
  **DK-2750 Ballerup (DK)**
- **STR B K, Dorte**
  **DK-2750 Ballerup (DK)**
- **JENSEN, Bo, Skaaning**
  **DK-2750 Ballerup (DK)**
- **PETERS, Dan**
  **DK-2750 Ballerup (DK)**

(56) References cited:

| | |
|---|---|
| EP-A- 0 520 200 | EP-A- 0 545 845 |
| EP-A- 0 604 353 | WO-A-91/18868 |
| WO-A-94/22807 | US-A- 4 004 016 |
| US-A- 5 190 976 | |

- **REIMLINGER H ET AL: "Synthesen mit heterocyclischen Aminen, X. Reaktionen einiger heterocyclischer Amine mit Propiolsäureester" CHEMISCHE BERICHTE, vol. 105, no. 3, 3 March 1972 (1972-03-03), pages 794-8, XP002119044**
- **CHEMICAL ABSTRACTS, vol. 78, no. 7, 19 February 1973 (1973-02-19) Columbus, Ohio, US; abstract no. 43360v, MEDVEDEVA M M ET AL: "Benzimidazole derivatives. XXIX. Synthesis of di(1-benzimidazolyl)alkanes and their relation to some nucleophilic agents" page 481; XP002119050 & KHIM. GETEROTSIKL. SOEDIN., no. 10, 1972, pages 1418-21,**
- **DATABASE CROSSFIRE [Online] Beilstein Institut für Literatur der organischen Chemie XP002119053 & KHIM. GETEROTSIKL. SOEDIN., vol. 18, no. 8, 1982 - 1086, page 8**
- **DATABASE CROSSFIRE [Online] Beilstein Institut für Literatur der organischen Chemie XP002119054 & KHIM. GETEROTSIKL. SOEDIN., no. 2, 1992, pages 190-3,**
- **CHEMICAL ABSTRACTS, vol. 69, no. 25, 16 December 1968 (1968-12-16) Columbus, Ohio, US; abstract no. 103313h, MIX H ET AL: "4-Guanidinobenzoic acid benzyl ester and 4-guanidinobenzoic acid 4'-nitrobenzylester: two new potent inhibitors of trypsin" page 9665; XP002119051 & HOPPE-SEYLER'S Z. PHYSIOL. CHEM., vol. 349, no. 9, 1968, pages 1237-8,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE CROSSFIRE [Online] Beilstein Institut für Literatur der organischen Chemie XP002119055 & ZH. OBSHCH. KHIM., vol. 32, 1962, page 2670
- STRUVE G E ET AL: "Syntheses of and structural assignments for some N-phosphono-2-iminoimidazolidines (cyclic guanidines)" JOURNAL OF ORGANIC CHEMISTRY, vol. 42, no. 25, 9 December 1977 (1977-12-09), pages 4035-40, XP002119045
- MCCONNAUGHIE A W ET AL: "Design and synthesis of RNA-specific groove-binding cations: implications for antiviral drug design" JOURNAL OF MEDICINAL CHEMISTRY, vol. 37, no. 8, 15 April 1994 (1994-04-15), pages 1063-9, XP002119046
- DATABASE CROSSFIRE [Online] Beilstein Institut für Literatur der organischen Chemie XP002119056 & J. CHEM. ENG. DATA, vol. 29, no. 1, 1984, pages 97-9,
- CHEMICAL ABSTRACTS, vol. 126, no. 19, 12 May 1997 (1997-05-12) Columbus, Ohio, US; abstract no. 257976j, CHAWLA S K ET AL: "Metal supported macrocycles: synthesis and characterization of some new 1,3-bis(benzimidazol-1-yl-methylene) benzene and 1,4-bis(benzimidazol-1-yl-methylene) benzene bridged metal(II) cation complexes" page 1162; XP002119052 & POLYHEDRON, vol. 16, no. 8, 1997, pages 1315-22,
- SHI Z ET AL: "Bridged dibenzimidazolinylidenes as new derivatives of tetraaminoethylene" TETRAHEDRON LETTERS, vol. 36, no. 16, 1995, pages 2741-4, XP002119047
- SHI Z ET AL: "N,N'-Bridged derivatives of 2,2'-bibenzimidazole" JOURNAL OF ORGANIC CHEMISTRY, vol. 60, no. 18, 8 September 1995 (1995-09-08), pages 5935-45, XP002119048
- ROSA J C ET AL: "Bis-quinolinium cyclophanes: 6,10-diaza-3(1,3),8(1,4)-dibenzena-1,5(1,4)-diquinolina-cyclodecaphane (UCL 1684), the first nanomolar, non-peptidic blocker of the apamin-sensitive Ca2+-activated K+ channel" JOURNAL OF MEDICINAL CHEMISTRY, vol. 41, no. 1, 1 January 1998 (1998-01-01), pages 2-5, XP002119049

## Description

### TECHNICAL FIELD

This invention relates to novel potassium channel blocking agents, and their use in the preparation of pharmaceutical compositions.

Moreover the invention is directed to pharmaceutical compositions useful for the treatment or alleviation of diseases or disorders associated with the activity of potassium channels, in particular asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary hearth disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer, and immune suppression.

### BACKGROUND ART

Ion channels are transmembrane proteins, which catalyse the transport of inorganic ions across cell membranes. The ion channels participate in processes as diverse as the generation and timing of action potentials, synaptic transmissions, secretion of hormones, contraction of muscles, etc.

All mammalian cells express potassium ($K^+$) channels in their cell membranes, and the channels play a dominant role in the regulation of the membrane potential. In nerve and muscle cells they regulate the frequency and form of the action potential, the release of neurotransmitters, and the degree of broncho- and vasodilation.

From a molecular point of view, the $K^+$ channels represent the largest and most diverse group of ion channels. For an overview they can be divided into five large subfamilies: Voltage-activated $K^+$ channels ($K_v$), long QT related $K^+$ channels (KvLQT), inward rectifiers ($K_{IR}$), two-pore $K^+$ channels ($K_{TP}$), and calcium-activated $K^+$ channels ($K_{ca}$).

The latter group, the $Ca^{2+}$-activated $K^+$ channels, consists of three well-defined subtypes: SK channels, IK channels and BK channels. SK, IK and BK refer to the single-channel conductance (Small, Intermediate and Big conductance K channel). The SK, IK, and BK channels exhibit differences in e.g. voltage- and calcium-sensitivity, pharmacology, distribution and function.

SK channels are present in many central neurons and ganglia, where their primary function is to hyperpolarize nerve cells following one or several action potentials, in order to prevent long trains of epileptogenic activity to occur. The SK channels are also present in several peripheral cells including skeletal muscle, gland cells, liver cells, and T-lymphocytes. The significance of SK channels in normal skeletal muscle is not clear, but their number is significantly increased in denervated muscle, and the large number of SK channels in the muscle of patients with myotonic muscle dystrophia, suggest a role in the pathogenesis of the disease.

Studies indicate that $K^+$ channels may be a therapeutic target in the treatment of a number of diseases including asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary hearth disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer, and immune suppression.

A number of neuromuscular blocking agents with effect on SK channels exist, e.g. apamin, atracurium, pancuronium and tubocurarine.

WO 97/48705 discloses a particular group of chemical compounds useful as calcium activated potassium channel blocking agents. However, their selectivity in respect of the SK channel is not disclosed.

US 5739127 and US 5760230 disclose other groups of chemical compounds acting on potassium channels.

### SUMMARY OF THE INVENTION

The present invention resides in the provision of novel chemical compounds capable of selectively blocking SK channels, or subtypes of SK channels.

Moreover the invention is directed to pharmaceutical compositions useful for the treatment or alleviation of diseases or disorders associated with the activity of potassium channels, including diseases or conditions like respiratory diseases such as asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary hearth disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer, and immune suppression.

Accordingly, in its first aspect, the invention provides novel chemical compounds of Formula I, identified below.

In another aspect, the invention provides pharmaceutical compositions comprising an effective amount of a chemical compound of the invention.

In further aspects the invention relates to the use of a chemical compound of the invention for the manufacture of a medicament for the treatment or alleviation of diseases or disorders associated with the activity of potassium channels.

## DETAILED DISCLOSURE OF THE INVENTION

### Potassium Channel Blocking Agents

In its first aspect, the invention provides novel chemical compounds. The chemical compounds of the invention is particularly useful as potassium channel blocking agents.

Thus, the invention provides a potassium channel blocking agent, in particular a SK channel blocking agent, being a bis(aminobenzimidazole) derivative of Formula I,

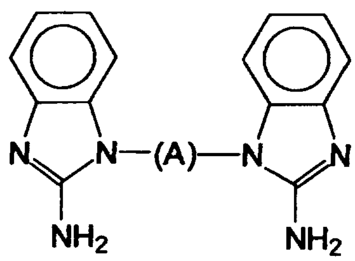

(I)

wherein

A represents a linear or branched alkylene chain having of from 1 to 15 carbon atoms, which alkylene group may be interrupted by one or more oxygen or sulphur atoms, or by one or more groups of the formula -NR'-, or =NR', wherein R' represents hydrogen or alkyl; or a radical of the formula $-(CH_2)_a-D-(CH_2)_b-$, wherein a and b, which may be identical or different, represent the number 0, 1, 2, 3, 4 or 5, and D represents a cycloalkyl group; provided that A is not $CH_2OCH_2$, $(CH_2)_{3-5}$, N=N or $(CH)_2CO$.

In a preferred embodiment, A is a radical of the formula $-(CH_2)_a-D-(CH_2)_b-$, wherein a and b, which may be identical or different, represent the number 0, 1, 2, 3, 4 or 5, and D represents a cycloalkyl group.

In another preferred embodiment A is a linear or branched alkylene chain having from 1 to 15 carbon atoms.

In an even more preferred embodiment A is decamethylene; octamethylene; hexamethylene; dimethylene; N,N'-dimethyl-diamino-methylene; N,N'-dimethyldiamino-dimethylene; N,N'-dimethyl-diamino-trimethylene; (cis and/or trans)-1,5-cyclooctylene; or (cis and/or trans)-1,3-dimethylcyclohexane-$\alpha,\alpha'$-diyl.

In a most preferred embodiment, the compound of Formula I is

1,3-Bis[(2-aminobenzimidazol-1-yl)methyl]cyclohexane;
1,6-Bis(2-aminobenzimidazol-1-yl)hexane;
1,2-Bis(2-aminobenzimidazol-1-yl)ethane; or
cis and/or trans-1,5-Bis(2-aminobenzimidazol-1-yl)cyclooctane.

Definition of Substituents

In the context of this invention a spacing group designates a substituent that links the two parts of the molecule and bring these parts into a relatively determined spatial inter-relationship. The spacing group may also be termed a linking group or a bridging group. The spacing group of the invention should link the two parts of the molecule in a not

too close and not too far distance from each another. It is currently believed that spacing groups comprising of from 2 to 20 atoms fulfil this requirement. Examples of such spacing groups are described herein, and summarised below.

| Spacing Group | Name |
|---|---|
| $-(CH_2)_{10}-$ | decamethylene; |
| $-(CH_2)_8-$ | octamethylene; |
| $-(CH_2)_6-$ | hexamethylene; |
| $-(CH_2)_2-$ | dimethylene; |
| $-N(CH_3)-CH_2-N(CH_3)-$ | N,N'-dimethyl-diamino-methylene; |
| $-N(CH_3)-CH_2-CH_2-N(CH_3)-$ | N,N'-dimethyl-diamino-dimethylene; |

| Spacing Group | Name |
|---|---|
| $-N(CH_3)-CH_2-CH_2-CH_2-N(CH_3)-$ | N,N'-dimethyl-diamino-trimethylene; |
| 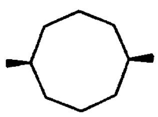 | (cis and/or trans)-1,5-cyclooctylene; |
| 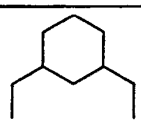 | (cis and/or trans)-1,3-dimethylcyclohexane-α,α'-diyl; |

In the context of this invention halogen represents a fluorine, a chlorine, a bromine or a iodine atom.

In the context of this invention an alkyl group designates a univalent saturated, straight or branched hydrocarbon chain. The hydrocarbon chain preferably contain of from one to eighteen carbon atoms ($C_{1-18}$-alkyl), more preferred of from one to six carbon atoms ($C_{1-6}$-alkyl; lower alkyl), including pentyl, isopentyl, neopentyl, tertiary pentyl, hexyl and isohexyl. In a preferred embodiment alkyl represents a $C_{1-4}$-alkyl group, including butyl, isobutyl, secondary butyl, and tertiary butyl. In a preferred embodiment of this invention alkyl represents a $C_{1-3}$-alkyl group, which may in particular be methyl, ethyl, propyl or isopropyl.

In the context of this invention a cycloalkyl group designates a cyclic alkyl group, preferably containing of from three to seven carbon atoms ($C_{3-7}$-cycloalkyl), including cyclopropyl, cyclobutyl, cyclopentyl, cyclohexyl and cycloheptyl.

In the context of this invention an alkoxy group designates an "alkyl-O-" group, wherein alkyl is as defined above.

In the context of this invention an alkoxy-alkyl group designates an "alkyl-O-alkyl-" group, wherein alkyl is as defined above.

In the context of this invention an amino group may be a primary ($-NH_2$), secondary (-NH-alkyl), or tertiary ($-N(alkyl)_2$) amino group, i.e. it may be substituted once or twice with an alkyl group as defined above.

In the context of this invention a mono- or polycyclic aryl group designates a monocyclic or polycyclic aromatic hydrocarbon group. Examples of preferred aryl groups of the invention are phenyl, naphthyl and anthracenyl.

In the context of this invention an aralkyl group designates a mono- or polycyclic aryl group as defined above, which aryl group is attached to an alkyl group as also defined above. An example of a preferred aralkyl group of the invention benzyl.

In the context of this invention a mono- or poly-heterocyclic group is a mono- or polycyclic compound, which holds one or more heteroatoms in its ring structure. One or more of the ring structures may in particular be aromatic (i.e. a heteroaryl). Preferred heterocyclic monocyclic groups of the invention are 5- or 6 membered heterocyclic monocyclic groups. Examples of preferred heterocyclic monocyclic groups of the invention are furanyl, imidazolyl, isothiazolyl, isoxazolyl, 1,2,3-oxadiazolyl, 1,2,4-oxadiazolyl, 1,2,5-oxadiazolyl, 1,3,4-oxadiazolyl, oxazolyl, pyrazinyl, pyrazolyl, pyridazinyl, pyridyl, pyrimidinyl, pyrrolyl, thiadiazolyl, thiazolyl, and thienyl. Examples of preferred heterocyclic polycyclic groups of the invention are benzimidazolyl, indolyl, isoquinolyl and quinolyl.

Also, in the context of this invention, a chemical compound comprising a tertiary amino group may also be made quaternary (quatemized) using an alkylation agent, in particular an alkyl halide, preferably the chloride, bromide or iodide of methyl or ethyl.

Specific Examples

In its most preferred embodiment, the chemical compound of the invention is one selected from Compounds 1a, 1b, 1e and 1j, described in the working examples and in Table 1, below.

**Table 1**
**Chemical Compounds of Formula I**

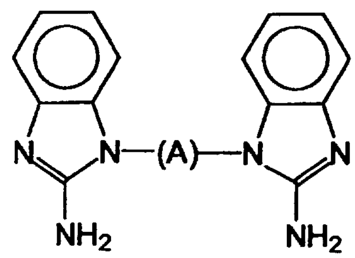

| Compound | A | Example |
|---|---|---|
| **1a*** | 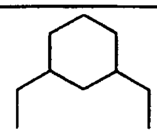 | 2 |
| **1b** | $-(CH_2)_6-$ | 1 |

| Compound | A | Example |
|---|---|---|
| 1e | $-(CH_2)_2-$ | 2 |
| 1j | 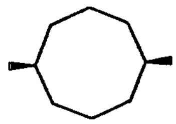 | 18 |

*cis/trans mixture

Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or l- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by *Jaques J, Collet A, & Wilen S* in "Enantiomers, Racemates, and Resolutions", John Wiley and Sons, New York (1981).

Moreover, some of the chemical compounds of the invention being oximes, may thus exist in two forms, syn- and anti-form (Z- and E-form), depending on the arrangement of the substituents around the -C=N- double bond. A chemical compound of the present invention may thus be the syn- or the anti-form (Z- and E-form), or it may be a mixture hereof.

Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulfonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the

succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Metal salts of a chemical compound of the invention include alkali metal salts, such as the sodium salt of a chemical compound of the invention containing a carboxy group.

The chemical compound of the invention may be provided in dissoluble or indissoluble forms together with a pharmaceutically acceptable solvents such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

**Methods of Preparation**

The chemical compounds of the invention may be prepared by conventional methods of chemical synthesis, e.g. those described in the working examples. The starting materials for the processes described in the present application are known or may readily be prepared by conventional methods from commercially available chemicals.

The end products of the reactions described herein may be isolated by conventional techniques, e.g. by extraction, crystallisation, distillation, chromatography, etc.

**Biological Activity**

The chemical compounds of the invention have been subjected to *in vitro* experiments and found particularly useful as potassium channel blocking agents. More particularly the compound of the invention are capable of selectively blockade of SK channels, e.g. SK1, SK2 and/or SK3 channels.

As described in the working examples, the compounds tested all showed a biological activity determined as $IC_{50}$ in the sub-micromolar and low micromolar range, i.e. of from below 1 to above 10 μM. Preferred compounds of the invention show a biological activity determined as described herein in the in the sub-micromolar and micromolar range, i.e. of from below 1 to about 100 μM.

Therefore, in another aspect, the invention relates to the use of a chemical compound of the invention for the manufacture of medicaments, which medicament may be useful for the treatment or alleviation of a disease or a disorder associated with the activity of potassium channels, in particular SK channels.

In a more preferred embodiment, the chemical compound of the invention may be use for the manufacture of medicaments for the treatment or alleviation of diseases or conditions like respiratory diseases such as asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary hearth disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer, and immune suppression.

**Pharmaceutical Compositions**

In yet another aspect the invention provides novel pharmaceutical compositions comprising a therapeutically effective amount of the chemical compound of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers and/or diluents.

In a preferred embodiment, the invention provides pharmaceutical compositions comprising the chemical compound of the invention, or a pharmaceutically acceptable salt or derivative thereof, together with one or more pharmaceutically acceptable carriers therefor and, optionally, other therapeutic and/or prophylactic ingredients. The carrier(s) must be "acceptable" in the sense of being compatible with the other ingredients of the formulation and not deleterious to the recipient thereof.

Pharmaceutical compositions of the invention may be those suitable for oral, rectal, nasal, topical (including buccal and sub-lingual), transdermal, vaginal or parenteral (including intramuscular, sub-cutaneous and intravenous) administration, or those in a form suitable for administration by inhalation or insufflation.

The chemical compound of the invention, together with a conventional adjuvant, carrier, or diluent, may thus

be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets; suppositories, and dispersible granules. A solid carrier can be one or more substances which may also act as diluents, favouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like

For topical administration to the epidermis the chemical compound according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert

base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient which ameliorates the symptoms or condition. Therapeutic efficacy and toxicity, e.g. $ED_{50}$ and $LD_{50}$, may be determined by standard pharmacological procedures in cell cultures or experimental animals. The dose ratio between therapeutic and toxic effects is the therapeutic index and may be expressed by the ratio $LD_{50}/ED_{50}$. Pharmaceutical compositions which exhibit large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The active ingredient may be administered in one or several doses per day. It is presently contemplated that compositions containing of from about 0.1 to about 500 mg of active ingredient per unit dosage, preferably of from about 1 to about 100 mg, most preferred of from about 1 to about 10 mg, are suitable for therapeutic treatments.

## EXAMPLES

The invention is further illustrated with reference to the following examples which are not intended to be in any way limiting to the scope of the invention as claimed.

**Example 1**

General reaction scheme for the syntheses of compounds **1a - 1f** (see Table 1)

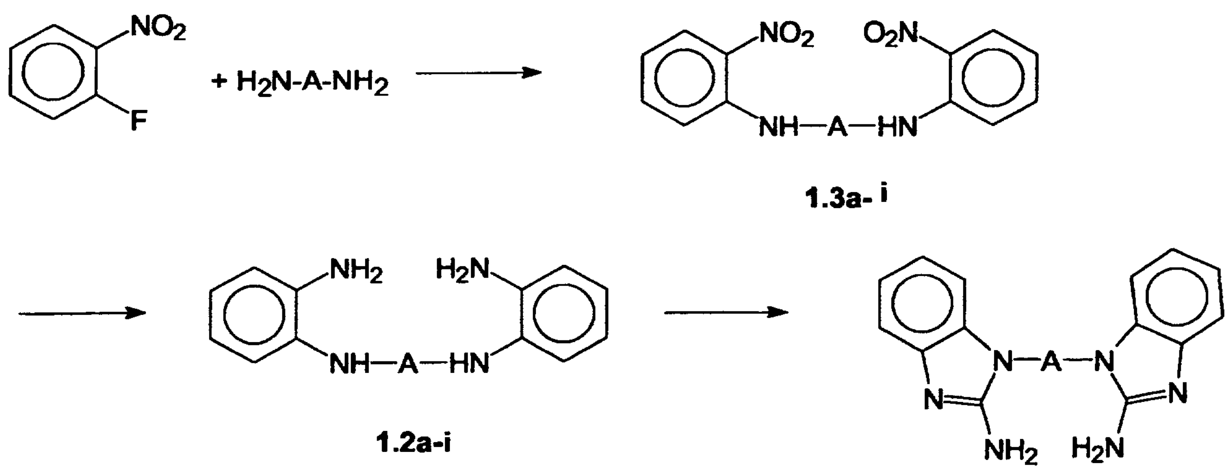

**1.3a- i**

**1.2a-i**

**1a-i**

1,4-Bis(2-aminobenzimidazol-1-yl)butane, 2HCl (Compound **1c).** A suspension of **1.2c**·2HCl (1.1 g, 3.2 mmol) in anhydrous DMF (10 ml) was wrapped in alu-foil to exclude light. A solution of cyanogen bromide (0.7 g, 6.6 mmol) in anhydrous DMF (5 ml) was added dropwise. The mixture was stirred in a nitrogen atmosphere at ambient temperature for three days, whereafter it was poured into ice-water. The precipitate was filtered off, washed with water and dried to leave **1c** (0.42 g). M.p. 292-294°C. 1,6-Bis(2-aminobenzimidazol-1-yl)hexane, 2HCl (Compound **1b)** was prepared analogously from **1.2b.** M.p. 145-147°C.

**Example 2**

1,3-Bis[(2-aminobenzimidazol-1-yl)methyl]cyclohexane (Compound **1a)** was prepared from **1.2a** as described in Example 1 with the following modifications: The solvent was anhydrous NMP. The reaction time was 24 hours. At the end of the reaction the mixture was poured into water and rendered alkaline by addition of aqueous sodium carbonate. The precipitate was filtered off and purified by column chromatography on silica gel using a mixture of dichloromethane, methanol and aqueous ammonia (9:1:0.1 v/v/v) as the eluent. Yield: 0.28 g (cis/trans mixture) which gradually decomposed upon melting.

1,2-Bis(2-aminobenzimidazol-1-yl)ethane (Compound **1e)** was prepared from **1.2e** in analogy with Example 2 using DMF as the solvent and a total of four equivalent of cyanogen bromide. The reaction time was 6 days. Yield: 0.13 g. M.p. 257-258°C.

**Example 3**

N,N'-Bis(2-aminophenyl)-1,4-butanediamine, 2HCl (Compound **1.2c**): To a suspension of 1.3c (1.2 g, 3.64 mmol) in a mixture of abs. EtOH and dichloromethane (50 ml, 9:1) was added Pd-catalyst (0.1 g, 5% Pd on activated carbon). The mixture was hydrogenated at ambient pressure until the $H_2$-uptake had ceased and thereafter filtered through celite. The filtrate was concentrated to a small volume under reduced pressure. Etheral hydrogen chloride was added, and the product was isolated by filtration. Yield: 1.14 g.

1,3-Bis(N-(2-aminophenyl)methylamine)cyclohexane, 2HCl (Compound **1.2a)** was prepared analogously from **1.3a.**

N,N'-Bis(2-aminoahenyl)-1,6-hexanediamine, 2HCl (Compound **1.2b**) was prepared analogously from **1.3b.**

N,N'-Bis(2-aminophenyl)ethylendiamine, 2HCl (Compound **1.2e**) was prepared analogously from **1.3e.**

**Example 4**

N,N'-Bis(2-nitrophenyl)-1,4-butanediamine (Compound **1.3c**): A mixture of 1,4-butanediamine (0.51 ml, 5.0 mmol), 1-fluoro-2-nitrobenzene (1.1 ml, 10.0 mmol) and triethylamine (1.39 ml, 10.0 mmol) in anhydrous DMF (5 ml) was heated to 100°C over-night. The cooled mixture was poured into ice-water. The product was filtered off, washed with water and dried to yield 1.22 g (24%).

1,3-Bis[N-(2-nitrophenyl)aminomethyl]cyclohexane (Compound **1.3a)** was prepared analogously from 1,3-bis (aminomethyl)cyclohexane.

N,N'-Bis(2-nitrophenyl)-1,6-hexanediamine (Compound **1.3b)** was prepared analogously from 1,6-hexanediamine.

N,N'-Bis(2-nitrophenyl)ethylenediamine (Compound **1.3e)** was prepared analogously from ethylenediamine.

**Example 5**

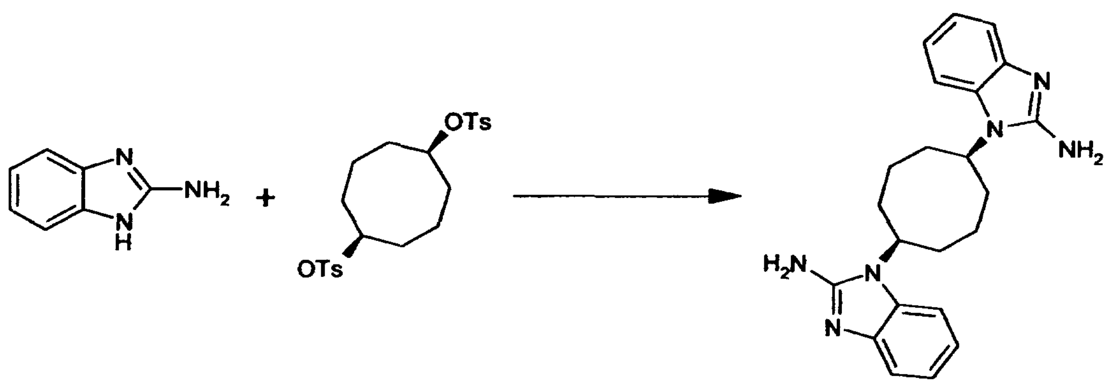

cis-1,5-bisi(2-amino-1-benzimidazolyl)cyclooctane (Compound 1j).

To a hot (60°C) solution of 2-aminobenzimidazole (0.22 g; 1.65 mmol) in DMF (25 ml) was added sodium hydride (70 mg; 60% dispersion in mineral oil), and the mixture was stirred for 30 minutes prior to addition of cis-1,5-bis(p-toluenesulfonyloxy) cyclooctane. The resulting mixture was stirred at 100°C for four days, cooled and filtered. The filtrate was diluted with water and the precipitate was filtered off, washed with water and dried to yield the desired product (9 mg).

**Example 6**

**Biological Activity**

This example demonstrates the biological activity of the compounds of the invention. Compound 1A of example 3, Compound 1B of example 2, and Compound 1J of example 19 were examined.

In this experiment, small-conductance $Ca^{2+}$-activated $K^+$ channels (SK channels, isoform 2) cloned from a rat cDNA library were stably expressed in HEK293 cells using standard procedures. The ionic current through the channels was recorded in the whole-cell mode of the patch-clamp technique.

Cells plated on coverslips are placed in a 15 µl perfusion chamber (flowrate ~1 ml/min), mounted on a IMT-2 microscope equipped with Nomarski or Hoffmann optics. The microscopes are placed on vibration-free tables in grounded Faraday cages.

All experiments are performed at room temperature (20 - 22°C). EPC-9 patch-clamp amplifiers (HEKA-electronics, Lambrect, Germany) are connected to Macintosh computers via ITC16 interfaces. Data are stored directly on the harddisk and analysed by the IGOR software according to the manufacturer's instructions.

The whole-cell configuration of the patch clamp technique is applied. Shortly described, the tip of a borosilicate pipette (resistance 2-4 MΩ) is gently (remote control system) placed on the cell membrane. Light suction results in a giga seal (pipette resistance increases to more than 1 GΩ), and the cell membrane is then ruptured by more powerful suction. Cell capacitance is electronically compensated and the resistance between the pipette and the cell interior (the series resistance, Rs) is measured and compensated for. Usually the cell capacitance ranges from 5 to 20 pF (depending on cell size), and the series resistance is in the range 3 to 6 MΩ. Rs-as well as capacitance compensation

are updated during the experiments (before each stimulus). All experiments with drifting Rs-values are discharged. Leak-subtractions are not performed.

Solutions

The extracellular (bath) solution contains (concentration in mM): 144 KCl, 2 $CaCl_2$, 1 $MgCl_2$, 10 HEPES (pH = 7.4).
Test compounds are dissolved as 1000 times concentrated stock solutions in DMSO, and then diluted in the extracellular solution.
In the experiments where the effect of channel activators is quantified (test 475), the intracellular (pipette) solution has the following composition (concentration in mM):
144 KCl, 10 EDTA, 1.4 $MgCl_2$, 5.17 $CaCl_2$, and 10 HEPES (pH = 7.2).
The calculated free concentration of $Ca^{2+}$ in this solution is 100 nM, and that of $Mg^{2+}$ is 1 mM. In these experiments, the concentration of $CaCl_2$ is 7.6 mM and that of $MgCl_2$ is 1.2 mM to give calculated free concentrations of 300 nM and 1 mM, respectively.

Quantification

After establishment of the whole-cell configuration, voltage-ramps (usually - 100 to + 100 mV) are applied to the cell every 5 sec. A stable baseline current is obtained within a period of 100-300 seconds and compounds are then added by changing to an extracellular solution containing the compound to be tested. Very little endogene current (<200 pA at 100 mV compared to 2-20 nA SK current) are activated under these circumstances in native HEK293 cells.
An $IC_{50}$ value is calculated from the kinetics of the block. The time-course of the decrease in current is fitted to the following equation:

$$I=I_0*(1-(C/C+(K_{off}/K_{on})))*(1-exp(-(C*K_{on}+K_{off})*t)))$$

where
$K_{off}$ = off-rate in $s^{-1}$
$K_{on}$ = on-rate in $M^{-1}s^{-1}$
$I_0$ = basal current in nA
C = drug concentration in $\mu M$
$IC_{50}$ equals the ratio $K_{off}/K_{on}$.

The compounds of the invention tested in this experiment all showed a biological activity determined as $IC_{50}$ in the sub-micromolar and low micromolar range, i.e. of from below 1 to above 10 $\mu M$.

## Claims

1. A chemical compound represented by the general formula I,

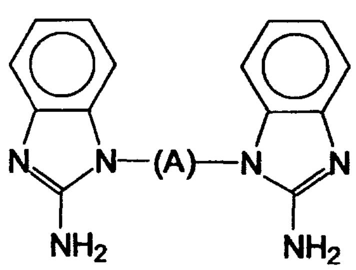

(I)

wherein
A represents a linear or branched alkylene chain having of from 1 to 15 carbon atoms, which alkylene group may be interrupted by one or more oxygen or sulphur atoms, or by one or more groups of the formula -NR'-, or =NR', wherein
R' represents hydrogen or alkyl; or

a radical of the formula $-(CH_2)_a-D-(CH_2)_b-$, wherein a and b, which may be identical or different, represent the number 0, 1, 2, 3, 4 or 5, and D represents a cycloalkyl group,
provided that A is not $CH_2OCH_2$, $(CH_2)_{3-5}$, N=N or $(CH)_2CO$.

**2.** The chemical compound of claim 1, wherein A is a radical of the formula $-(CH_2)_a-D-(CH_2)_b-$, wherein a and b, which may be identical or different, represent the number 0, 1, 2, 3, 4 or 5, and D represents a cycloalkyl group.

**3.** The chemical compound of claim 1, wherein A is a linear or branched alkylene chain having from 1 to 15 carbon atoms.

**4.** The chemical compound of claim 1, wherein A is
decamethylene; octamethylene; hexamethylene; dimethylene; N,N'-dimethyldiamino-methylene; N,N'-dimethyl-diamino-dimethylene; N,N'-dimethyl-diaminotrimethylene; (cis and/or trans)-1,5-cyclooctylene; or (cis and/or trans)-1,3-dimethylcyclohexane-α,α'-diyl.

**5.** The chemical compound of claim 1, being
1,3-Bis[(2-aminobenzimidazol-1-yl)methyl]cyclohexane;
1,6-Bis(2-aminobenzimidazol-1-yl)hexane;
1,2-Bis(2-aminobenzimidazol-1-yl)ethane; or
cis-1,5-bis(2-amino-1-benzimidazolyl)cyclooctane.

**6.** A pharmaceutical composition comprising an effective amount of a chemical compound according to claims 1-5.

**7.** Use of the chemical compound of claims 1-5 for the manufacture of a medicament for the treatment or alleviation of diseases or disorders associated with the activity of potassi um channels.

**8.** The use according to claim 7, wherein the disease or disorder is asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic hearth disease, angina pectoris, coronary hearth disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer, and immune suppression.

**Patentansprüche**

**1.** Chemische Verbindung, wiedergegeben durch die allgemeine Formel I,

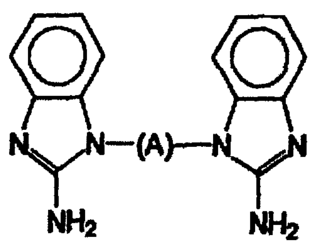

(I)

worin
A bedeutet eine lineare oder verzweigte Alkylenkette mit 1 bis 15 Kohlenstoffatomen, wobei die Alkylengruppe durch ein oder mehrere Sauerstoff- oder Schwefelatome oder durch eine oder mehrere Gruppen der Formel -NR'- oder =NR', worin R' Wasserstoff oder Alkyl bedeutet, unterbrochen sein kann, oder einen Rest der Formel $-(CH_2)_a-D-(CH_2)_b-$, worin a und b, die gleich oder verschieden sein können, die Zahl 0, 1, 2, 3, 4 oder 5 bedeuten, und D eine Cycloalkylgruppe bedeutet, mit der Maßgabe, dass A nicht $CH_2OCH_2$, $(CH_2)_{3-5}$, N=N oder $(CH)_2CO$ ist.

2. Chemische Verbindung nach Anspruch 1, worin A ein Rest der Formel $-CH_2)_a-D-(CH_2)_b-$ ist, worin a und b, die gleich oder verschieden sein können, die Zahl 0, 1, 2, 3, 4 oder 5 bedeuten, und D eine Cycloalkylgruppe bedeutet.

3. Chemische Verbindung nach Anspruch 1, worin A eine lineare oder verzweigte Alkylengruppe mit 1 bis 15 Kohlenstoffatomen ist.

4. Chemische Verbindung nach Anspruch 1, worin A Decamethylen, Octamethylen, Hexamethylen, Dimethylen, N, N'-Dimethyldiaminomethylen, N,N'-Dimethyldiaminodimethylen, N,N'-Dimethyldiaminotrimethylen, (cis und/oder trans)-1,5-Cyclooctylen oder (cis und/oder trans)-1,3-Dimethylcyclohexan-α,α'-diyl ist.

5. Chemische Verbindung nach Anspruch 1, welche
1,3-Bis[(2-aminobenzimidazol-1-yl)methyl]cyclohexan,
1,6-Bis(2-aminobenzimidazol-1-yl)hexan,
1,2-Bis(2-aminobenzimidazol-1-yl)ethan oder
cis-1,5-Bis(2-amino-1-benzimidazolyl)cyclooctan ist.

6. Pharmazeutische Zusammensetzung, umfassend eine wirksame Menge einer chemischen Verbindung gemäß den Ansprüchen 1 bis 5.

7. Verwendung der chemischen Verbindung nach den Ansprüchen 1 bis 5 zur Herstellung eines Medikaments zur Behandlung oder Erleichterung von Krankheiten oder Störungen, die mit der Aktivität von Kaliumkanälen verbunden sind.

8. Verwendung gemäß Anspruch 7, worin die Krankheit oder Störung Asthma, zystische Fibrose, chronisch-obstruktive Lungenerkrankung und Rhinorrhea, Krämpfe, Gefäßspasmen, Koronararterienspasmen, Nierenstörungen, polyzystische Nierenerkrankung, Blasenspasmen, Urininkontinenz, Blasenausflussverschluss, irritables Darmsyndrom, gastrointestinale Störung, sekretorische Diarrhoe, Ischämie, Zerebralischämie, ischämische Herzerkrankung, Angina pectoris, koronare Herzerkrankung, traumatische Hirnverletzung, Psychose, Angstzustände, Depression, Demenz, Erinnerungs- und Aufmerksamkeitsschwäche, Alzheimersche Krankheit, Dysmenorrhoe, Narkolepsie, Reynaudsche Krankheit, Claudicatio intermittens, Sjorgrensches Syndrom, Migräne, Arrhythmie, Bluthochdruck, Absenzen, myotonische Muskeldystrophie, Xerostomie, Diabetes Typ II, Hyperinsulinämie, vorzeitige Wehen, Kahlheit, Krebs und Immununterdrückung ist.

## Revendications

1. Composé chimique représenté par la formule générale I,

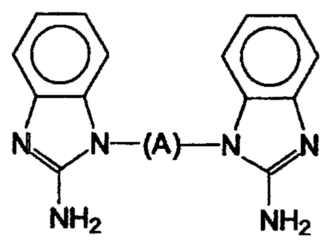

(I)

dans laquelle
A représente une chaîne alkylène linéaire ou ramifiée ayant de 1 à 15 atomes de carbone, ce groupe alkylène pouvant être interrompu par un ou plusieurs atomes d'oxygène ou de soufre, ou par un ou plusieurs groupes de formule -NR'-, ou =NR', où R' représente un hydrogène ou un alkyle ; ou
un radical de formule $-(CH_2)_a-D-(CH_2)_b-$, où a et b, qui peuvent être identiques ou différents, représentent le nombre 0, 1, 2, 3, 4 ou 5, et D représente un groupe cycloalkyle,
à la condition que A ne soit pas $CH_2OCH_2$, $(CH_2)_{3-5}$, N=N ou $(CH)_2CO$.

2. Composé chimique selon la revendication 1, dans lequel A est un radical de formule $-(CH_2)_a-D-(CH_2)_b-$, où a et b, qui peuvent être identiques ou différents, représentent le nombre 0, 1, 2, 3, 4 ou 5, et D représente un groupe

cycloalkyle.

3. Composé chimique selon la revendication 1, dans lequel A est une chaîne alkylène linéaire ou ramifiée ayant de 1 à 15 atomes de carbone.

4. Composé chimique selon la revendication 1, dans lequel A est un groupe décaméthylène ; octaméthylène ; hexaméthylène ; diméthylène ; N,N'-diméthyl-diaminométhylène ; N,N'-diméthyl-diamino-diméthylène ; N,N'-diméthyl-diamino-triméthylène ; (cis et/ou trans)-1,5-cyclooctylène ; ou (cis et/ou trans)-1,3-diméthylcyclohexane-$\alpha,\alpha'$-diyle.

5. Composé chimique selon la revendication 1, qui est
le 1,3-bis[(2-aminobenzimidazol-1-yl)méthyl]cyclohexane ;
le 1, 6-bis(2-aminobenzimidazol-1-yl)hexane ;
le 1,2-bis(2-aminobenzimidazol-1-yl)éthane ; ou
le cis-1,5-bis(2-amino-1-benzimidazolyl)cyclooctane.

6. Composition pharmaceutique comprenant une quantité efficace d'un composé chimique selon les revendications 1 à 5.

7. Utilisation du composé chimique selon les revendications 1 à 5 pour la fabrication d'un médicament pour le traitement ou l'atténuation de maladies ou de troubles associés à l'activité des canaux potassiques.

8. Utilisation selon la revendication 7, dans laquelle la maladie ou le trouble est un asthme, une mucoviscidose, une bronchopneumopathie chronique obstructive et une rhinorrhée, des convulsions, des spasmes vasculaires, des spasmes coronariens, des troubles rénaux, une maladie polykystique des reins, des spasmes de la vessie, une incontinence urinaire, une obstruction du vidage de la vessie, un syndrome du côlon irritable, un dysfonctionnement gastrointestinal, une diarrhée sécrétoire, une ischémie, une ischémie cérébrale, une cardiopathie ischémique, une angine de poitrine, une maladie coronarienne, une lésion cérébrale traumatique, une psychose, une anxiété, une dépression, une démence, une déficience de la mémoire et de l'attention, une maladie d'Alzheimer, une dysménorrhée, une narcolepsie, une maladie de Reynaud, une claudication intermittente, un syndrome de Sjorgren, une migraine, une arythmie, une hypertension, des absences, une dystrophie musculaire myotonique, une xérostomie, un diabète de type II, une hyperinsulinémie, un travail prématuré, une calvitie, un cancer et une suppression immunitaire.